# EUROPEAN PATENT APPLICATION

(11) **EP 2 476 758 A1**
(43) Date of publication of application: **18.07.2012**
(21) Application number: 10813839.7
(22) Date of filing: 07.09.2010
(51) Int. Cl.: C12Q 1/68, A61K 31/7056, A61K 38/21, G01N 33/53, G01N 37/00, A61P 1/16

(54) **METHOD FOR PREDICTION OF THERAPEUTIC EFFECT ON CHRONIC HEPATITIS C**

(30) Priority: 07.09.2009 JP 2009229977
(71) Applicant: Murakami, Yoshiki, Kyoto-shi, Kyoto 606-8501 (JP)
(72) Inventor: Murakami, Yoshiki, Kyoto-shi, Kyoto 606-8501 (JP)
(74) Representative: Cohausz & Florack
(86) International application number: PCT/JP2010/065306
(87) International publication number: WO 2011/027893

(57) **Abstract**

A method for predicting therapeutic effect of combination therapy with peginterferon and ribavirin in chronic hepatitis C therapy, comprising:
a drug-sensitivity marker detection step of detecting a drug-sensitivity marker in a biological sample; and
a drug-sensitivity evaluation step of evaluating drug sensitivity of the biological sample on the basis of an expression level of the drug-sensitivity marker, wherein
the biological sample is a cell or tissue derived from liver, and
the drug-sensitivity marker comprises at least one microRNA selected from the group consisting of microRNAs shown in SEQ ID NOs: 1 to 37.

## Description

### [Technical Field]

The present invention relates to a method for predicting therapeutic effect on chronic hepatitis C.

### [Background Art]

The prevalence of hepatitis C virus infection in the world is approximately 3%. Infection with hepatitis C virus leads to chronic hepatic disease (chronic hepatitis or hepatic cirrhosis) at a high rate, and eventually to hepatic cancer (NPL 1). A current standard strategy of chronic hepatitis C therapy is the combination therapy with peginterferon and ribavirin. However, the efficacy ratio of the combination therapy in patients infected with hepatitis C virus genotype lb, with which many Japanese patients are infected, is as low as about 50%. A reason for this is adverse effects of the combination therapy (NPL 2). For this reason, development of a new strategy of chronic hepatitis C therapy has been desired.
microRNAs are small RNA molecules which are not translated into proteins, and regulate gene expression by binding to messenger RNAs in a base-sequence-specific manner. microRNAs are deeply associated with cell development and the like (NPLs 3 and 4). In particular, microRNA-122 (hereinafter referred to as miR-122) accounts for 70% of the microRNAs in the liver (NPL 5). It is known that administration of interferon beta results in change in expression levels of microRNAs in cells. Of these microRNAs, 8 microRNAs have a possibility of controlling replication of hepatitis C virus (NPL 6). It has been reported so far that some microRNAs suppress replication of hepatitis C virus (NPLs 6 to 8). miR-122 is considered to be associated with interferon response at an early stage of interferon therapy (NPL 9).

Predictions of effect of interferon therapy have been attempted so far. In the cases of infection with hepatitis C virus genotype lb, the presence or absence of mutations in the base sequence of the interferon sensitivity-determining region (ISDR) influences the interferon therapy (NPL 10). In addition, it is also reported that the presence or absence of mutations of the 70th amino acid and the 91st amino acid in the region, called the core, of hepatitis C virus influences the interferon therapy (NPL 11).

Exhaustive gene expression analyses using microarrays are useful for diagnosis of diseases and prediction of drug effects. In particular, such analyses are reported for chronic rheumatoid arthritis (NPL 12), systemic lupus erythematosus (NPL 13), and the like. In addition, regarding malignant diseases, analyses using microarrays are reported for diagnosis of breast cancer, the presence or absence of distant metastasis (NPLs 14 and 15), pre-treatment evaluation as to whether or not certain drugs are effective for target diseases (NPLs 16 and 17), and the like.

### [Citation List]

### [Non Patent Literature]

[NPL 1] Annu Rev Pathol 2006; 1: 23-61
[NPL 2] N Engl J Med 2002; 347: 975-82
[NPL 3] Nature 2004; 431: 350-5
[NPL 4] Trends Genet 2007; 23: 243-9
[NPL 5] Curr Biol 2002; 12: 735-9
[NPL 6] Nature 2007; 449: 919-22
[NPL 7] Science 2005; 309: 1577-81
[NPL 8] J Hepatol 2009; 50: 453-60
[NPL 9] Nat Med 2009; 15: 31-3
[NPL 10] N Engl J Med 1996; 334: 77-81
[NPL 11] Intervirology 2007; 50: 361-8
[NPL 12] Ann Rheum Dis 2004; 63: 1387-92
[NPL 13] J Immunol 2002; 169: 5-9
[NPL 14] N Engl J Med 2006; 355: 560-9
[NPL 15] Science 1999; 286: 531-7
[NPL 16] Proc Natl Acad USA 2001; 98: 10787-92
[NPL 17] PLoS Genet 2008; 4: e1000287
[NPL 18] Genome Biol 2002; 3: RESEARCH0036
[NPL 19] Bioinformatics 2003; 19: 1090-9

### [Summary of Invention]

### [Technical Problem]

The present invention has been made in view of such circumstances, and an object of the present invention is to provide a novel method for predicting therapeutic effect of combination therapy with peginterferon and ribavirin on chronic hepatitis C.

### [Solution to Problem]

The present inventors have made earnest studies to achieve the above object. As a result, the present inventors have found that expression of specific microRNAs is strongly correlated with therapeutic effect of combination therapy with peginterferon and ribavirin on chronic hepatitis C patients. This finding has lead to the completion of the present invention.

Specifically, the present inventionprovides a method for predicting therapeutic effect of combination therapy with peginterferon and ribavirin in chronic hepatitis C therapy, comprising:
a step of detecting a drug-sensitivity marker in a biological sample; and
a step of evaluating drug sensitivity of the biological sample on the basis of an expression level of the drug-sensitivity marker, wherein
the biological sample is a cell or tissue derived from liver, and
the drug-sensitivity marker comprises at least one microRNA selected from the group consisting of miR-18a (SEQ ID NO: 1), miR-145 (SEQ ID NO: 2), miR-34b (SEQ ID NO: 3), miR-331 (SEQ ID NO: 4), let-7d (SEQ ID NO: 5), miR-200a (SEQ ID NO: 6), miR-200b (SEQ ID NO: 7), miR-652 (SEQ ID NO: 8), miR-132 (SEQ ID NO: 9), miR-143 (SEQ ID NO: 10), miR-425-5p (SEQ ID NO: 11), miR-185 (SEQ ID NO: 12), miR-141 (SEQ ID NO: 13), miR-200c (SEQ ID NO: 14), miR-195 (SEQ ID NO: 15), miR-411 (SEQ ID NO: 16), miR-10a (SEQ ID NO: 17), miR-345 (SEQ ID NO: 18), miR-214 (SEQ ID NO: 19), miR10b (SEQ ID NO: 20), miR-377 (SEQ ID NO: 21), let-7b (SEQ ID NO: 22), miR-182 (SEQ ID NO: 23), miR-429 (SEQ ID NO: 24), miR-107 (SEQ ID NO: 25), miR-127 (SEQ ID NO: 26), miR-500 (SEQ ID NO: 27), miR-376a (SEQ ID NO: 28), miR-199a* (SEQ ID NO: 29), miR-218 (SEQ ID NO: 30), miR-125a (SEQ ID NO: 31), miR-106b (SEQ ID NO: 32), miR-199a (SEQ ID NO: 33), miR-27b (SEQ ID NO: 34), miR-422b (SEQ ID NO: 35), miR-122a (SEQ ID NO: 36), and miR-23b (SEQ ID NO: 37).

In addition, in a preferred embodiment, the present invention provides the above-described method, wherein a method for detecting the drug-sensitivity marker in the step of detecting a drug-sensitivity marker is microarray analysis.

Moreover, in another preferred embodiment, the present invention provides the above-described method, where in a method for evaluating the drug-sensitivity marker in the step of evaluating drug sensitivity is Monte Carlo cross validation.

### [Advantageous Effects of Invention]

The present invention makes it possible to predict therapeutic effect of combination therapy with peginterferon and ribavirin in chronic hepatitis C therapy in a simple and convenient manner, by using the expression level of the drug-sensitivity marker as an index. In particular, the microRNA expression pattern analysis developed by the present inventors makes it possible to determine the effectiveness of the combination therapy with peginterferon and ribavirin with a high reliability before the therapy. In addition, by analyzing microRNA expression patterns, it is possible to provide a route to understand pathology, such as progression, of hepatitis.

Predictions of effect of chronic hepatitis C therapy have been attempted from the past by using virus genotype, virus amount, degree of liver fibrosis, age, body weight, race, fat content in liver tissue, or the like as an index. In addition, since the period of chronic hepatitis C therapy is 48 weeks, determination as to whether or not this therapy is effective is also attempted during the therapy. According to a prediction, cases where viruses in blood disappear within 12 weeks after initiation of the therapy will result in sustained virologic response (SVR) at a probability of 60%. As mentioned above, mutations in the interferon sensitivity-determining region and mutations of the 70th and 91st amino acids in the core region are also used as indices of prediction of therapeutic effect on chronic hepatitis C. It was found that the method of the present invention using microRNA expression as an index had an accuracy of 70.5%, when a prediction of the effect was conducted by using Monte Carlo cross validation. This accuracy is higher than those of the conventional methods. The method of the present invention enabling more accurate examination is highly advantageous, in consideration of the facts that the combination therapy with peginterferon and ribavirin requires a treatment period of as long as 48 weeks, entails considerable costs (60000 yen or more per month in a case of a general health insurance where the patient should pay 30% of the costs), and is ineffective for cases of about 50% of patients because of development of adverse effects.

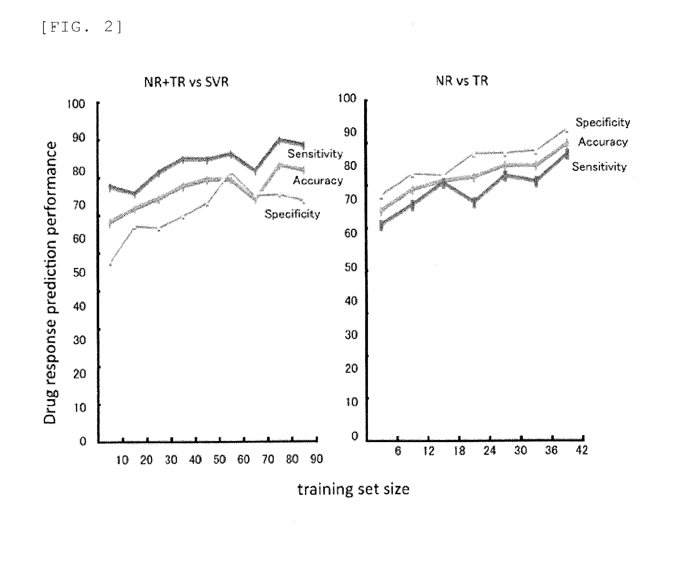

In addition, when Monte Carlo cross validation is used in the evaluation of drug sensitivity in the method of the present invention, the accuracy rises, as the number of analyses increases (see Fig. 2). Hence, a further high precision can be expected, when a large-scale sample analysis is conducted.

In general, a microarray analysis is capable of simultaneously analyzing expression of approximately 20000 human genes. Studies using various kinds of clinical information and microarray analyses in combination have been conducted so far. However, there was such a drawback that, when the number of target genes is large, it is impossible to conduct sufficient analysis, unless the number of samples required for the analysis is increased. The number of human microRNAs known at present is 706, and the number of genes to be subj ected to the analysis is smaller than that in the case of the conventional exhaustive human gene analysis. For this reason, the analysis of microRNAs with a microarray is advantageous, because sufficient analysis can be conducted even with a small number of samples. Another advantage of the analysis of microRNAs with a microarray is that expression levels of microRNAs do not greatly vary among individuals. Such advantages are brought about, when microarray analysis is employed for the detection of microRNA expression in the method of the present invention.

### [Brief Description of Drawings]

[Fig. 1] Fig. 1 shows graphs showing analysis results of microRNA expression.
[Fig. 2] Fig. 2 shows graphs showing clinical results of a method for predicting therapeutic effect of the present invention.

### [Description of Embodiments]

As described above, the present invention is a method for predicting therapeutic effect of combination therapy with peginterferon and ribavirin in chronic hepatitis C therapy, comprising:
a step of detecting a drug-sensitivity marker in a biological sample; and
a step of evaluating drug sensitivity of the biological sample on the basis of an expression level of the drug-sensitivity marker, wherein
the biological sample is a cell or tissue derived from liver, and
the drug-sensitivity marker comprises at least one microRNA selected from the group consisting of miR-18a (SEQ ID NO: 1), miR-145 (SEQ ID NO: 2), miR-34b (SEQ ID NO: 3), miR-331 (SEQ ID NO: 4), let-7d (SEQ ID NO: 5), miR-200a (SEQ ID NO: 6), miR-200b (SEQ ID NO: 7), miR-652 (SEQ ID NO: 8), miR-132 (SEQ ID NO: 9), miR-143 (SEQ ID NO: 10), miR-425-5p (SEQ ID NO: 11), miR-185 (SEQ ID NO: 12), miR-141 (SEQ ID NO: 13), miR-200c (SEQ ID NO: 14), miR-195 (SEQ ID NO: 15), miR-411 (SEQ ID NO: 16), miR-10a (SEQ ID NO: 17), miR-345 (SEQ ID NO: 18), miR-214 (SEQ ID NO:19), miR10b (SEQ ID NO: 20), miR-377 (SEQ ID NO: 21), let-7b (SEQ ID NO: 22), miR-182 (SEQ ID NO: 23), miR-429 (SEQ ID NO: 24), miR-107 (SEQ ID NO: 25), miR-127 (SEQ ID NO: 26), miR-500 (SEQ ID NO: 27), miR-376a (SEQ ID NO: 28), miR-199a* (SEQ ID NO: 29), miR-218 (SEQ ID NO: 30), miR-125a (SEQ ID NO: 31), miR-106b (SEQ ID NO: 32), miR-199a (SEQ ID NO: 33), miR-27b (SEQ ID NO: 34), miR-422b (SEQ ID NO: 35), miR-122a (SEQ ID NO: 36), and miR-23b (SEQ ID NO: 37).

### <Step of Detecting Drug-sensitivity marker>

A method for detecting the drug-sensitivity marker in the step of detecting a drug-sensitivity marker is not particularly limited, and conventionally known methods can be used. However, the method is preferably microarray analysis.

### <Step of Evaluating Drug Sensitivity>

A method for evaluating the drug-sensitivity marker in the step of evaluating drug sensitivity is not particularly limited, and conventionally known methods can be used. However, the method is preferably Monte Carlo cross validation.

### <Biological Sample>

The biological sample only needs to be a cell or tissue derived from liver, and the other aspects thereof are not particularly limited.

### <Drug-sensitivity marker>

The drug-sensitivity marker only needs to comprise at least one microRNA selected from the group consisting of miR-18a (SEQ ID NO: 1), miR-145 (SEQ ID NO: 2), miR-34b (SEQ ID NO: 3), miR-331 (SEQ ID NO: 4), let-7d (SEQ ID NO: 5), miR-200a (SEQ IDNO: 6), miR-200b (SEQ ID NO: 7), miR-652 (SEQ ID NO: 8), miR-132 (SEQ ID NO: 9), miR-143 (SEQ ID NO: 10), miR-425-5p (SEQ ID NO: 11), miR-185 (SEQ ID NO: 12), miR-141 (SEQ ID NO: 13), miR-200c (SEQ ID NO: 14), miR-195 (SEQ ID NO: 15), miR-411 (SEQ ID NO: 16), miR-10a (SEQ ID NO: 17), miR-345 (SEQ ID NO: 18), miR-214 (SEQ ID NO: 19), miR10b (SEQ ID NO: 20), miR-377 (SEQ ID NO: 21), let-7b (SEQ ID NO: 22), miR-182 (SEQ ID NO: 23), miR-429 (SEQ ID NO: 24), miR-107 (SEQ ID NO: 25), miR-127 (SEQ ID NO: 26), miR-500 (SEQ ID NO: 27), miR-376a (SEQ ID NO: 28), miR-199a* (SEQ IDNO: 29) , miR-218 (SEQ ID NO: 30) , miR-125a (SEQ ID NO: 31), miR-106b (SEQ ID NO: 32), miR-199a (SEQ ID NO: 33), miR-27b (SEQ ID NO: 34), miR-422b (SEQ ID NO: 35), miR-122a (SEQ ID NO: 36), and miR-23b (SEQ ID NO: 37), and the other aspects thereof are not particularly limited.

### [Examples]

Hereinafter, the present invention will be described based on Examples. However, the present invention is not limited to Examples.

### (Example 1)

### (Analysis subject patients)

Analysis subjects were 99 chronic hepatitis C patients (infected with genotype 1b) (Table 1). It was checked that these patients did not suffer from autoimmune hepatitis, alcoholic liver disease, hepatitis B, or HIV infection. In addition, it was checked that these patients had not received interferon therapy or therapy using an immunosuppressant so far. Serum HCV RNA was measured by using Amplicor-HCV monitor (manufactured by Roche). The inflammation and fibrosis of liver tissue were evaluated by using the Metavir classification (J Hepatol 1996; 25: 649-554, Am J Clin Pathol 2000; 114: 712-718). Before interferon therapy, values of AST, ALT, total bilirubin, ALP, Y-GTP, and hemoglobin in blood, white blood cell count, and platelet count were measured. This clinical study was approved by Kyoto University Graduate School and Faculty of Medicine, Ethics Committee, who found no ethical problems in this clinical study, and the contents of this clinical study were in accordance with the Declaration of Helsinki.

**[Table 1]**

| Characteristics | SVR (n=46) | TR (n=28) | NR (n=25) | P-value |
|---|---|---|---|---|
| Age (year) | 57.0 ± 9.8 | 61.2 ± B.8 | 60.6 ± 7.6 | 0.09† |
| Male (% | 28 (61%) | 11 (39%) | 9 (36%) | 0.08§ |
| Body weight (kg) | 59.5 ± 9.0 | 56.6 ± 9.9 | 56.0 ± 7.7 | 0.13† |
| HCV RNA (x10*) coples/ml) | 1.90 ± 1.95 | 1.83 ± 1.04 | 1.58 ± 0.93 | 0.62† |
| Fibrosis stage | | | | |
| F 0 | 1 | 1 | 1 | 0.50§ |
| F 1 | 29 | 16 | 10 | |
| F 2 | 10 | 7 | 6 | |
| F 3 | 6 | 4 | 7 | |
| F 4 | 0 | 0 | 1 | |
| WBC (x10³/mm³) | 5.31 ± 1.59 | 5.18 ± 1.24 | 4.71 ± 1.15 | 0.29† |
| Hemoglobin (g/dl) | 14.:2 ± 1.26 | 13.6 ± 1.35 | 13.5 ± 1.13 | 0.022† |
| Platelet (x10⁶/mm³) | 16.7 ± 5.0 | 16.4 ± 4.0 | 15.2 ± 6.1 | 0.25† |
| AST (IU/L) | 54.8 ± 48.1 | 46.6 ± 29.3 | 57.0 ± 28.5 | 0.17† |
| ALT (IU/L) | 74.5 ± 87.8 | 47.9 ± 28.6 | 67.6 ± 43.2 | 0.15† |
| γGTP (IU/L) | 56.0 ± 69.4 | 38.5 ± 28.9 | 74.3 ± 59.0 | 0.025† |
| ALP [IU/L] | 248 ± 71.5 | 245 ± 75.7 | 323 ± 151 | 0.038† |
| Total bilirubin (mg/dl) | 0.67 ± 0.22 | 0.72 ± 0.30 | 0.68 ± 0.19 | 0.95† |
| Albumin (g/dl) | 4.21 ± 0.31 | 4.13 ± 0.27 | 4.01 ± 0.48 | 0.14† |

### (Therapeutic Strategy and Therapeutic Effect Determination)

Peginterferon (pegylated interferon alfa-2b; manufactured by Schering-Plough Corporation) and ribavirin (manufactured by Schering-Plough Corporation) were administered for 48 weeks. Peginterferon was administered at a dose of 1 µg/kg weight once a week. Ribavirin was administered at a dose recommended by Schering-Plough Corporation. The therapeutic effect was determined by being classified into the following three groups.

SVR (sustained virologic response); cases where HCV RNA in blood was not detectable for 6 months after termination of the administration of peginterferon and ribavirin.

TR (transient response); cases where HCV RNA in blood was not detectable immediately after the termination of the administration of peginterferon and ribavirin, but HCV RNA in blood became detectable within 6 months after the termination.

NR (no response): cases where HCV RNA in blood was continuously detectable during the course.

### (Method for extracting total RNA)

Liver tissue was collected from patients by liver biopsy, and the collected tissue was immersed in an RNA Later solution (manufactured by Ambion), and stored at -80 °C. The total RNA was collected by using a mirVana miRNA Isolation Kit (manufactured by Ambion).

### (Microarray for microRNAs)

Analysis was conducted by using a microarray for microRNAs (manufactured by Agilent Technologies). By using Human microRNA Microarray kit (manufactured by Agilent Technologies), 100 ng of the total RNA extracted by the above-described method was labeled, and hybridized with the array (bound according to base sequences) according to the version 1.5 manual. To detect hybridized and labeled RNAs, a G2505B scanner (manufactured by Agilent Technologies) was used. The information obtained here was quantified and analyzed by using Agilent feature extraction software (v 9.5.3.1) (manufactured by Agilent Technologies).

### (Method for Calculating Therapeutic Effect Prediction)

The calculation was conducted by using Monte Carlo cross validation (NPLs 18 and 19).

### (Method for Detecting Mutations in Amino Acids in Core Region of Chronic Hepatitis C Virus and Mutations in Base Sequence in Interferon Sensitivity-Determining Region)

Predictions of effect of interferon therapy have been attempted so far. In the cases of infection with chronic hepatitis C virus genotype 1b, the presence or absence of mutations in the base sequence in the interferon sensitivity-determining region (ISDR) influences the interferon therapy (NPL 10). In addition, it is also reported that the presence or absence of mutations of the 70th amino acid and the 91st amino acid in the region, called the core, of chronic hepatitis C virus influences the interferon therapy (NPL 11).

### (Statistical Treatment)

Analyses between any two groups were conducted by using Student's t test. In addition, analyses between the three groups were conducted by Kruskal-Wallis test and Fisher's exact test. Cases where P<0.01 or less were determined to have significant differences.

### [Example 1]

A MicroRNA expression analysis was conducted on liver tissue of 99 chronic hepatitis C patients by using a microRNA microarray. MicroRNA expression patterns were analyzed for the individual groups (SVR, TR, and NR), which were classified based on the therapeutic effect in the combination therapy with peginterferon and ribavirin. microRNAs whose expression was different between the SVR group and the NR group were identified.

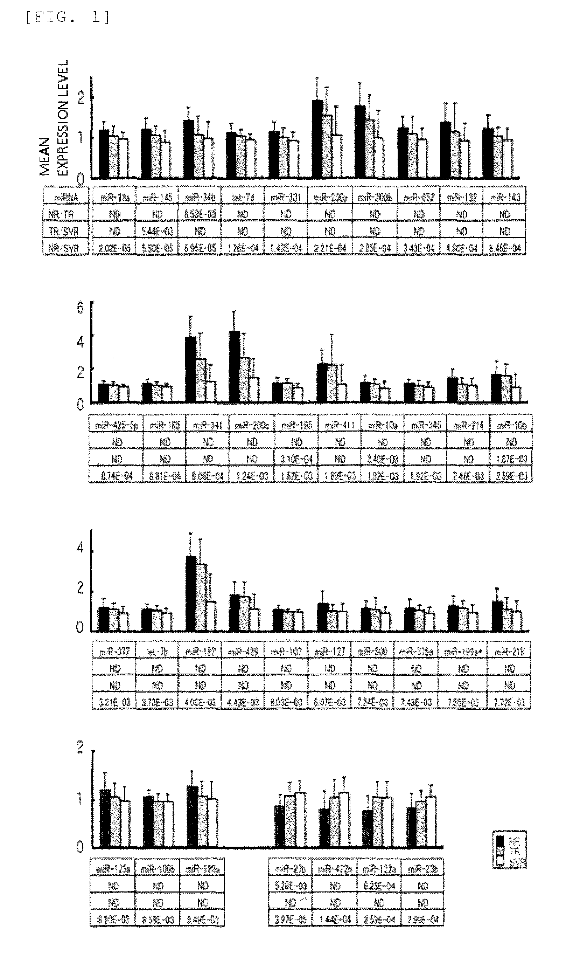

Fig. 1 shows microRNAs whose expression levels were different among the groups classified based on the therapeutic effect. In this figure, the vertical axis represents the relative microRNA expression level, and variations of the expression level of each microRNA are expressed in the form of ±1 standard deviation. In addition, the expression level of each microRNA was analyzed by Student's t test, and those with p<0.01 or less were employed. As a result of these analysis, 4 microRNAs were found whose expression levels in a group increased as the effectiveness of the interferon therapy in the group increased, and 33 microRNAs were found whose expression levels decreased in a reversed manner. Note that the effectiveness of the interferon therapy was higher in the order of NR<TR<SVR. From these, it has been found that those microRNAs are microRNAs which vary according to the effectiveness of the combination therapy with peginterferon and ribavirin.

### [Example 2]

By using the expression patterns of microRNAs, a prediction was attempted as to whether or not therapy subject patients would respond to the therapy, before the combination therapy with peginterferon and ribavirin was conducted.

Fig. 2 shows results thereof. Expression patterns of 35 microRNAs were analyzed by using Monte Carlo cross validation. The left graph shows SVR and non-SVR (TR+NR). While 80 were set for a training set, a prediction was made by using the remaining 19 biological samples. As a result, the SVR and the non-SVR were distinguishable from each other with an accuracy of 70.5%, a specificity of 63.3%, and a sensitivity of 76.8%. The right graph shows an attempt to predict TR and NR. A prediction was conducted, while 42 were set for a training set. As a result, the accuracy was 70.0%, the specificity was 73.7%, and the sensitivity was 67.5%. From the results described above, it has been found that the method for predicting therapeutic effect of the present invention makes it possible to diagnose effective cases and ineffective cases of the combination therapy with peginterferon and ribavirin with a high reliability, before the combination therapy with peginterferon and ribavirin is conducted.

### [Comparative Example 1]

To demonstrate the usefulness of the method for predicting therapeutic effect of the present invention, comparative examination was conducted between the prediction method of the present invention and conventionally used methods for predicting therapeutic effect. The presence or absence of mutations in the interferon sensitivity-determining region of hepatitis C virus present in blood has a relationship with prediction of effect of the combination therapy with peginterferon and ribavirin, where the interferon therapy will be effective for those having mutations on the base sequence in the interferon sensitivity-determining region, and will not be effective for those having no mutations (NPL 10).

Tables 2 to 5 show the presence or absence of mutations in the interferon sensitivity-determining region in 80 cases. Those having no mutations are indicated by "W", those having one or two mutations are indicated by "IM", and those having three or more mutations are indicated by "M" (Table 3 to 5). When the prediction method of the present invention was used regarding the index, the accuracy was 56.6%, the specificity was 50.0%, and the sensitivity was 60.6% (Table 2).

Mutations of amino acids in the core region of hepatitis C virus present in blood have a relationship with prediction of effect of the combination therapy with peginterferon and ribavirin. In a case (M) where a mutation is present in the 70th amino acid in the core region, no therapeutic effect is expected. In also a case (M) where a mutation is present in the 91st amino acid in the core region, no therapeutic effect is expected. In a case (W) where no mutation is present in the 70th amino acid in the core region, the virus will respond to the therapy. In also a case (W) where no mutation is present in the 91st amino acid, the virus will respond to the therapy (NPL 11). The prediction method of the present invention was used regarding this index. As a result, the accuracy was 62.5%, the specificity was 86.5%, and the sensitivity was 41.9% for the 70th amino acid; whereas the accuracy was 47.5%, the specificity was 78.4%, and the sensitivity was 20.9% for the 91st amino acid (Table 2). In Tables 3 to 5, cases where no mutation of the 70th amino acid was present are indicated by "R", and cases where no mutation of the 91st amino acid was present are indicated by "L". Those indicated by other letters all had mutations.

**[Table 2]**

| | Accuracy (%) | Sensitivity (%) | Specificity (%) |
|---|---|---|---|
| miRNA | 70.5 | 76.8 | 63.3 |
| ISDR | 56.6 | 60.0 | 50.0 |
| Core 70aa | 62.5 | 41.9 | 86.5 |
| Core 91aa | 47.5 | 20.9 | 78.4 |

**[Table 3]**

| code No. | Result | ISDR | Core | Core-70 | Core-91 |
|---|---|---|---|---|---|
| OCH-105 | NR | IM | WW | R | L |
| OCH-111 | NR | IM | WW | R | L |
| OCH.118 | NR | W/IM | MW | R | M |
| OCH-119 | NR | W | MW | R | M |
| OCH-122 | NR | IM | MW | Q | L |
| OCH-123 | NR | W | MW | Q | M |
| OCH-126 | NR | W | MW | Q | L |
| OCH-127 | NR | ND | ND | ND | ND |
| OCH-132 | NR | W | WW | R | L |
| OCH-137 | NR | IM | MW | Q | L |
| OCH-140 | NR | W | WW | R | L |
| OCH-142 | NR | W | WW | R | L |
| OCR-144 | NR | IM | WW | R | L |
| OCH-145 | NR | ND | ND | ND | ND |
| OCH-192 | NR | ND | ND | ND | ND |
| OCH-204 | NR | IM/M | MW | Q | L |
| OCH-205 | NR | W | MW | Q | L |
| OCR- 206 | NR | ND | ND | ND | ND |
| OCH-207 | NR | W | MM | Q | M |
| OCH-208 | NR | W/IM | MM | Q | M |
| OCH-209 | NR | W/IM | MW | Q | L |
| OCH-210 | NR | ND | ND | ND | ND |
| OCH-211 | NR | ND | MW | Q | L |
| OCH-22 | NR | IM | WW | R | L |
| OCH-242 | NR | ND | ND | ND | ND |

**[Table 4]**

| code No- | Result | ISGR | Core | Core-70 | Core-91 |
|---|---|---|---|---|---|
| OCH-101 | TR | W | WM | R | M |
| OCH-102 | TR | ND | ND | ND | ND |
| OCH-106 | TR | IM | MW | Q | L |
| OCH-118 | TR | IM | WM | R | L |
| OCH-115 | TR | M | MW | Q | L |
| OCH-117 | TR | M | WM | R | M |
| OCH-120 | TR | W | WM | R | M |
| OCH-125 | TR | W | MW | Q | L |
| OCH-128 | TR | IM | WW | R | L |
| OCH-129 | TR | W | WW | R | L |
| OCH-133 | TR | W/IM | MW | Q | L |
| OCH-129 | TR | IM | WW | R | L |
| OCH-135 | TR | W | WW | R | L |
| OCH-141 | TR | W | WW | R | L |
| OCH-151 | TR | ND | ND | ND | ND |
| OCH-152 | TR | W | WM | R | M |
| OCH-159 | TR | ND | WW | R | L |
| OCH-166 | TR | ND | WW | R | L |
| OCH-213 | TR | W | WW | R | L |
| OCH-214 | TR | W/IM | WM | R | M |
| OCH- 215 | TR | W | WW | R | L |
| OCH-216 | TR | ND | ND | ND | ND |
| OCH-217 | TR | W | WW | R | L |
| OCH-216 | TR | W/IM | WW | R | L |
| OCH-219 | TR | W/ IM | MW | Q | L |
| OCH-220 | TR | W | MM | H | M |
| OCH-221 | TR | W | WW | R | L |
| OCH-239 | TR | ND | ND | ND | ND |

**[Table 5]**

| code No- | Result | ISDR | Core | Core-70 | Core-91 |
|---|---|---|---|---|---|
| OCH-103 | SVR | W | MW | H | L |
| OCH-104 | SVR | W | WW | R | L |
| OCH-107 | SVR | IM | WW | R | L |
| OCH-108 | SVR | W | WW | R | L |
| OCH-109 | SVR | W | WW | R | L |
| OCH-110 | SVR | W/IM | MM | H | M |
| OCH-112 | SVR | IM | WW | R | L |
| OCH-114 | SVR | M | WW | R | L |
| OCH-116 | SVR | IM | WW | R | L |
| OCH-121 | SVR | ND | ND | ND | ND |
| OCH-124 | SVR | W | WW | R | L |
| OCH-130 | SVR | IM/M | WW | R | L |
| OCH-131 | SVR | IM/M | WW | R | L |
| OCH-136 | SVR | W/IM | MW | H | L |
| OCH-138 | SVR | ND | ND | ND | ND |
| OCH-139 | SVR | W | WM | R | M |
| OCH-143 | SVR | ND | ND | ND | ND |
| OCH-150 | SVR | W | WW | R | L |
| OCH-153 | SVR | ND | ND | ND | ND |
| OCH-154 | SVR | W/IM | WW | R | L |
| OCH-155 | SVR | IM | WW | R | L |
| OCH-156 | SVR | ND | WW | R | L |
| OCH-157 | SVR | ND | WW | R | L |
| OCH-158 | SVR | ND | WW | R | L |
| OCH-160 | SVR | W/IM | WW | R | L |
| OCH-186 | SVR | ND | WW | R | L |
| OCH-187 | SVR | ND | MW | H/Q | L |
| OCH-189 | SVR | ND | WW | R | L |
| OCH-190 | SVR | ND | WM | R | M |
| OCH-191 | SVR | ND | WW | R | L |
| OCH-194 | SVR | ND | ND | ND | ND |
| OCH-195 | SVR | ND | WW | R | L |
| OCH-222 | SVR | W | WM | R | M |
| OCH-228 | SVR | ND | WW | R | L |
| OCH-229 | SVR | M | WW | R | L |
| OCH-230 | SVR | W/IM | WW | R | L |
| OCH-231 | SVR | ND | WM | R | M |
| OCH-232 | SVR | ND | WM | R | M |
| OCH-233 | SVR | W | WM | R | M |
| OCH-234 | SVR | IM | WM | R | M |
| OCH-236 | SVR | W | WW | R | L |
| OCH-237 | SVR | IM | MW | Q | L |
| OCH-238 | SVR | ND | ND | ND | ND |
| OCH-240 | SVR | ND | ND | ND | ND |
| OCH-241 | SVR | ND | ND | ND | ND |
| OCH-242 | SVR | ND | ND | ND | ND |

### [Industrial Applicability]

According to the method for predicting therapeutic effect of the present invention, the detection of an expression level of a microRNA as a drug-sensitivity marker in a biological sample makes it possible to estimate, for example, the effectiveness of the combination therapy with peginterferon and ribavirin with a high reliability, and hence to select an appropriate therapeutic strategy. From the viewpoint of therapeutic effect, it is important to change the contents of therapy depending on the status of a disease among individuals, even when the disease is the same. The present invention can be expected to be applied to personalized medicine.

In addition, the present invention has revealed that expression patterns of microRNAs vary among therapeutic effects. A novel gene therapy using this is conceivable. For example, the combination therapy with peginterferon and ribavirin may be made effective by enhancing the expression of microRNAs whose expression is low in no response (NR).

Moreover, expression patterns of microRNAs of chronic hepatitis C serve as useful information for understanding how abnormal microRNA expression participates in progress of hepatitis. Hence, the present invention may contribute to development of a novel strategy of hepatitis therapy.

## Claims

1. A method for predicting therapeutic effect of combination therapy with peginterferon and ribavirin in chronic hepatitis C therapy, comprising:
a step of detecting a drug-sensitivity marker in a biological sample; and
a step of evaluating drug sensitivity of the biological sample on the basis of an expression level of the drug-sensitivity marker, wherein
the biological sample is a cell or tissue derived from liver, and
the drug-sensitivity marker comprises at least one microRNA selected from the group consisting of miR-18a (SEQ ID NO: 1), miR-145 (SEQ ID NO: 2), miR-34b (SEQ ID NO: 3), miR-331 (SEQ ID NO: 4), let-7d (SEQ ID NO: 5), miR-200a (SEQ ID NO: 6), miR-200b (SEQ ID NO: 7), miR-652 (SEQ ID NO: 8), miR-132 (SEQ ID NO: 9), miR-143 (SEQ ID NO: 10), miR-425-5p (SEQ ID NO: 11) , miR-185 (SEQ ID NO: 12) , miR-141 (SEQ ID NO: 13), miR-200c (SEQ ID NO: 14), miR-195 (SEQ ID NO: 15), miR-411 (SEQ ID NO: 16), miR-10a (SEQ ID NO: 17), miR-345 (SEQ ID NO: 18), miR-214 (SEQ ID NO: 19), miR10b (SEQ ID NO: 20), miR-377 (SEQ ID NO: 21), let-7b (SEQ ID NO: 22), miR-182 (SEQ ID NO: 23), miR-429 (SEQ ID NO: 24), miR-107 (SEQ ID NO: 25), miR-127 (SEQ ID NO: 26), miR-500 (SEQ ID NO: 27), miR-376a (SEQ ID NO: 28), miR-199a* (SEQ ID NO: 29), miR-218 (SEQ ID NO: 30), miR-125a (SEQ ID NO: 31), miR-106b (SEQ ID NO: 32), miR-199a (SEQ ID NO: 33), miR-27b (SEQ ID NO: 34), miR-422b (SEQ ID NO: 35), miR-122a (SEQ ID NO: 36), and miR-23b (SEQ ID NO: 37).

2. The method according to claim 1, wherein a method for detecting the drug-sensitivity marker in the step of detecting a drug-sensitivity marker is microarray analysis.

3. The method according to claim 1 or 2, wherein a method for evaluating the drug-sensitivity marker in the step of evaluating drug sensitivity is Monte Carlo cross validation.


SEQUENCE LISTING

<110> MURAKAMI Yoshiki
<120> METHOD FOR PREDICTING THERAPEUTIC EFFECT ON CHRONIC
HEPATITIS C
<130> XXPF10-531WO
<150> JP2009-229977
<151> 2009-09-07
<160> 37
<170> PatentIn version 3.1

<210> 1
<211> 23
<212> RNA
<213> Homo sapiens
<400> 1
uaaggugcau cuagugcaga uag 23

<210> 2
<211> 22
<212> RNA
<213> Homo sapiens
<400> 2
ggauuccugg aaauacuguu cu 22

<210> 3
<211> 23
<212> RNA
<213> Homo sapiens
<400> 3
uaggcagugu cauuagcuga uug 23

<210> 4
<211> 22
<212> RNA
<213> Homo sapiens
<400> 4
cuagguaugg ucccagggau cc 22

<210> 5
<211> 22
<212> RNA
<213> Homo sapiens
<400> 5
agagguagua gguugcauag uu 22

<210> 6
<211> 22
<212> RNA
<213> Homo sapiens
<400> 6
uaacacuguc ugguaacgau gu 22

<210> 7
<211> 22
<212> RNA
<213> Homo sapiens
<400> 7
uaauacugcc ugguaaugau ga 22

<210> 8
<211> 21
<212> RNA
<213> Homo sapiens
<400> 8
aauggcgcca cuaggguugu g 21

<210> 9
<211> 22
<212> RNA
<213> Homo sapiens
<400> 9
uaacagucua cagccauggu cg 22

<210> 10
<211> 21
<212> RNA
<213> Homo sapiens
<400> 10
ugagaugaag cacuguagcu c 21

<210> 11
<211> 8
<212> RNA
<213> Homo sapiens
<400> 11
aaugacac 8

<210> 12
<211> 22
<212> RNA
<213> Homo sapiens

<400> 12
uggagagaaa ggcaguuccu ga 22

<210> 13
<211> 22
<212> RNA
<213> Homo sapiens
<400> 13
uaacacuguc ugguaaagau gg 22

<210> 14
<211> 23
<212> RNA
<213> Homo sapiens
<400> 14
uaauacugcc ggguaaugau gga 23

<210> 15
<211> 21
<212> RNA
<213> Homo sapiens
<400> 15
uagcagcaca gaaauauugg c 21

<210> 16
<211> 21
<212> RNA
<213> Homo sapiens
<400> 16
uaguagaccg uauagcguac g 21

<210> 17
<211> 23
<212> RNA
<213> Homo sapiens
<400> 17
uacccuguag auccgaauuu gug 23

<210> 18
<211> 22
<212> RNA
<213> Homo sapiens
<400> 18
gcugacuccu aguccagggc uc 22

<210> 19

<211>  22
<212>  RNA
<213>  Homo sapiens
<400>  19
acagcaggca cagacaggca gu                                                22

<210>  20
<211>  23
<212>  RNA
<213>  Homo sapiens
<400>  20
uacccuguag aaccgaauuu gug                                               23

<210>  21
<211>  22
<212>  RNA
<213>  Homo sapiens
<400>  21
aucacacaaa ggcaacuuuu gu                                                22

<210>  22
<211>  22
<212>  RNA
<213>  Homo sapiens
<400>  22
ugagguagua gguugugugg uu                                                22

<210>  23
<211>  24
<212>  RNA
<213>  Homo sapiens
<400>  23
uuuggcaaug guagaacuca cacu                                              24

<210>  24
<211>  22
<212>  RNA
<213>  Homo sapiens
<400>  24
uaauacuguc ugguaaaacc gu                                                22

<210>  25
<211>  23
<212>  RNA
<213>  Homo sapiens
<400>  25

agcagcauug uacagggcua uca 23

<210> 26
<211> 22
<212> RNA
<213> Homo sapiens
<400> 26
ucggauccgu cugagcuugg cu 22

<210> 27
<211> 23
<212> RNA
<213> Homo sapiens
<400> 27
uaauccuugc uaccugggug aga 23

<210> 28
<211> 21
<212> RNA
<213> Homo sapiens
<400> 28
aucauagagg aaaauccacg u 21

<210> 29
<211> 8
<212> RNA
<213> Homo sapiens
<400> 29
uacaguag 8

<210> 30
<211> 21
<212> RNA
<213> Homo sapiens
<400> 30
uugugcuuga ucuaaccaug u 21

<210> 31
<211> 24
<212> RNA
<213> Homo sapiens
<400> 31
ucccugagac ccuuuaaccu guga 24

<210> 32
<211> 21

<212>  RNA
<213>  Homo sapiens
<400>  32
uaaagugcug acagugcaga u                                                21

<210>  33
<211>  23
<212>  RNA
<213>  Homo sapiens
<400>  33
cccaguguuc agacuaccug uuc                                              23

<210>  34
<211>  21
<212>  RNA
<213>  Homo sapiens
<400>  34
uucacagugg cuaaguucug c                                                21

<210>  35
<211>  8
<212>  RNA
<213>  Homo sapiens
<400>  35
cuggacuu                                                               8

<210>  36
<211>  8
<212>  RNA
<213>  Homo sapiens
<400>  36
uggagugu                                                               8

<210>  37
<211>  21
<212>  RNA
<213>  Homo sapiens
<400>  37
aucacauugc cagggauuac c                                                21